(19) Europäisches Patentamt
European Patent Office
Office européen des brevets

(11) Publication number: **0 155 096**
**B1**

(12) **EUROPEAN PATENT SPECIFICATION**

(45) Date of publication of patent specification: **04.10.89**

(51) Int. Cl.[4]: **C 07 J 73/00,** A 61 K 31/435

(21) Application number: **85301122.9**

(22) Date of filing: **20.02.85**

(60) **Divisional application 88119105.0 filed on 20/02/85.**

(54) **17 Beta-Substituted-4-aza-5-alpha-androstenones and their use as 5-alpha-reductase inhibitors.**

(30) Priority: **27.02.84 US 584062**
**27.02.84 US 584061**

(43) Date of publication of application:
**18.09.85 Bulletin 85/38**

(45) Publication of the grant of the patent:
**04.10.89 Bulletin 89/40**

(84) Designated Contracting States:
**AT BE CH DE FR GB IT LI LU NL SE**

(56) References cited:
**EP-A-0 004 949**

**CHEMICAL ABSTRACTS, vol. 95, no. 13, 28th September 1981, page 99, abstract no. 109055j, Columbus, Ohio, US; T. LIANG et al.: "Inhibition of 5alpha-reductase, receptor binding, and nuclear uptake of androgens in the prostate by a 4-methyl-4-aza-steroid", & J. BIOL. CHEM. 1981, 256(15), 7998-8005**

(73) Proprietor: **MERCK & CO. INC.**
**126, East Lincoln Avenue P.O. Box 2000**
**Rahway New Jersey 07065-0900 (US)**

(72) Inventor: **Rasmusson, Gary H.**
**155 Park Place**
**Watchung New Jersey 07060 (US)**
Inventor: **Reynolds, Glenn F.**
**252 Edgewood Avenue**
**Westfield New Jersey 07090 (US)**

(74) Representative: **Crampton, Keith John Allen et al**
**D YOUNG & CO 10 Staple Inn**
**London WC1V 7RD (GB)**

(56) References cited:
**JOURNAL OF ORGANIC CHEMISTRY, vol. 46, no. 7, July 1981, pages 1442-1446, American Chemical Society, Washington, D.C., US; T.G. BACK: "Oxidation of azasteroid lactams and alcohols with benzeneseleninic anhydride"**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 155 096 B1**

## Description

The present invention is concerned with 17β-substituted-4-aza-5α-androstenones, their preparation, and their use as testosterone-5α-reductase inhibitors.

It is well known that certain undesirable physiological manifestations, such as acne vulgaris, seborrhoea, female hirsutism, and male pattern baldness and benign prostatic hypertrophy, are the result of hyperandrogenic stimulation caused by an excessive accumulation of testosterone or similar androgenic hormones in the metabolic system. Early attempts to provide a chemotherapeutic agent to counter the undesirable results of hyperandrogenicity resulted in the discovery of several steroidal antiandrogens having undesirable hormonal activities of their own. The oestrogens, for example, not only counteract the effect of the androgens but have a feminizing effect as well. Non-steroidal antiandrogens have also been developed, for example, 4'-nitro-3'-trifluoromethylisobutyranilide [See Neri et al., Endo., Vol. 91, No. 1 (1972)]. However, these products, though devoid of hormonal effects, are peripherally active, competing with the natural androgens for receptor sites, and hence have a tendency to feminize a male host or the male foetus of a female host.

It more recently became known that the principal mediator of androgenic activity in some target organs is 5α-dihydrotestosterone, and that this is formed locally in the target organ by the action of testosterone-5α-reductase. It was therefore postulated and has been demonstrated that inhibitors of testosterone-5α-reductase serve to prevent or lessen symptoms of hyperandrogenic stimulation. Nayfe *et al.,* [Steroids, 14, 269 (1969)] demonstrated that methyl 4-androsten-3-one-17β-carboxylate was a testosterone-5α-reductase inhibitor *in vitro.* Then Voigt and Hsia, Endocrinology, 92, 1216 (1973), Canadian Patent No. 970,692, demonstrated that the above ester and the parent free acid, 4-androsten-3-one-17β-carboxylic acid are both active inhibitors of testosterone-5α-reductase, *in vitro.* They further demonstrated that topical application of either testosterone of 5α-dihydrotesterone caused enlargement of the female hamster flank organ, an androgen-dependent sebaceous structure. However, concomitant administration of 4-androsten-3-one-17β-carboxylic acid or its methyl ester inhibited the response elicited by testosterone but did not inhibit the response elicited by 5α-dihydrotestosterone. These results were interpreted as indicating that the compounds were antiandrogenic by virtue of their ability to inhibit testosterone-5α-reductase.

A number of 4-aza steroid compounds are known. See, for example, U.S. Patent Specifications Nos. US—A—2,227,876, 3,239,417, 3,264,301 and 3,285,918; French Patent Specification No. FR—A—1,465,544; Doorenbos and Solomons, J. Pharm. Sci. 62, 4, pp. 638—640 (1973); Doorenbos and Brown, J. Pharm. Sci., 60, 8, pp. 1234—1235 (1971); and Doorenbos and Kim, J. Pharm. Sci. 63, 4, pp. 620—622 (1974).

European Patent Specification EP—A—0 004 949 discloses *inter alia* that androstenone compounds of the formula:

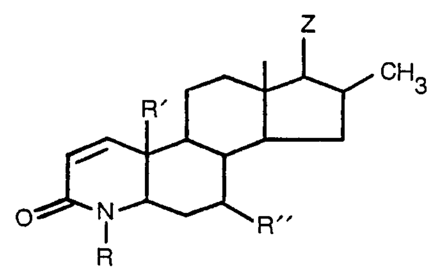

wherein R is hydrogen, methyl or ethyl; R' is hydrogen or methyl; R'' is hydrogen or β-methyl; and Z is —NHCO—($C_{1-12}$ alkyl) or —CO—$NR^9R^{10}$, in which each of $R^9$ and $R^{10}$, independently of the other, is hydrogen, $C_{1-4}$ straight or branched alkyl or $C_{3-6}$ cycloalkyl; are active as testosterone 5α-reductase inhibitors.

In addition U.S. Patent Specifications Nos. US—A—4,377,584 and 4,220,775 of Rasmusson *et al.* describe a group of 4-aza-17β-substituted-5α-androstan-3-ones that are said to be useful in the treatment of hyperandrogenic conditions.

However, none of the documents mentioned above suggests that any of the novel 17β-N-(monosubstituted carbamoyl)-4-aza-5α-androsten-1-en-3-ones of the present invention as defined below would be expected to have utility as highly potent testosterone-5α-reductase inhibitors.

The present invention provides novel 17β-N-(monosubstituted carbamoyl)-4-aza-5α-androsten-1-en-3-one compounds of the formula:

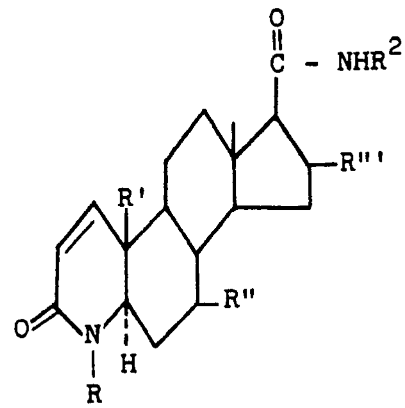

I

in which R is hydrogen, methyl or ethyl;

$R^2$ is a tertiary alkyl radical containing up to 12 carbon atoms and having a tertiary carbon atom directly bonded to the carbamoyl nitrogen atom;

R′ is hydrogen or methyl;

R″ is hydrogen or β-methyl; and

R‴ is hydrogen, α-methyl or β-methyl.

Preferred compounds have the formula:

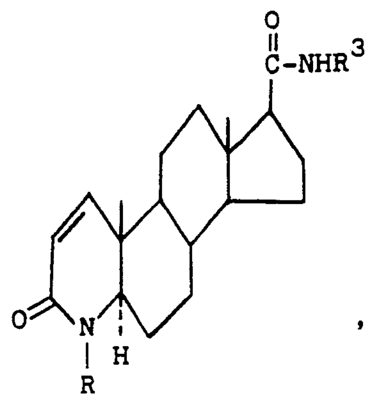

II

in which R is hydrogen, methyl or ethyl, and $R^3$ is tertiary alkyl of from 4—8 carbons and having a tertiary carbon atom directly bonded to the carbamoyl nitrogen atom.

Representative compounds of the present invention include the following:

17β-(N-*t*-butylcarbamoyl)-4-aza-4-methyl-5α-androst-1-en-3-one;
17β-(N-isobutylcarbamoyl)-4-aza-4-methyl-5α-androst-1-en-3-one;
17β-(N-*t*-octylcarbamoyl)-4-aza-4-methyl-5α-androst-1-en-3-one;
17β-(N-1,1-diethylbutyl-carbamoyl)-4-aza-4-methyl-5α-androst-1-en-3-one;
17β-(N-neopentylcarbamoyl)-4-aza-4-methyl-5α-androst-1-en-3-one;
17β-(N-*t*-amylcarbamoyl)-4-aza-4-methyl-5α-androst-1-en-3-one;
17β-(N-*t*-hexylcarbamoyl)-4-aza-4-methyl-5α-androst-1-en-3-one;

and the corresponding compounds in which the 4-methyl substituent is replaced by a hydrogen atom or an ethyl radical.

The novel compounds of the present invention are prepared from the known steroid ester of the formula:

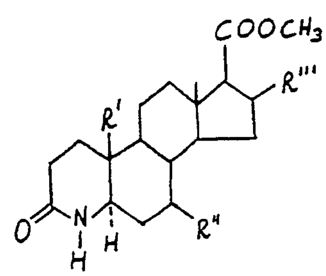

III

viz., 17β-(methoxycarbonyl)-4-aza-5α-androstan-3-one, or another 17β-alkoxycarbonyl-4-aza-5α-androstan-3-one compound, which may have the indicated substitution indicated by R′, R″ and R‴, by (A) dehydrogenating the starting material to produce the corresponding compound containing a double-bond

in the 1,2-position of the A-ring (B), converting the 17-substituent into an N-monosubstituted carbamoyl substituent, and optionally (C) alkylating the A-ring nitrogen to introduce a 4-methyl or 4-ethyl substituent in the A ring. In carrying out the process, it is essential that Stage (A) dehydrogenation of the 1,2-position of the steroid A ring be carried out using a 4-aza-5α-androstane-3-one-compound having no substituent other than hydrogen attached to the A-ring nitrogen. Stage (B) may consist of one or more chemical steps and if desired may take place before stage (A) or following stage (A) or stage (C).

In a practical method of carrying out this process, the product I are formed by (1) heating a 17β-alkoxycarbonyl-4-aza-5α-androstan-3-one compound III with a dehydrogenating agent such as benzeneseleninic anhydride in refluxing chlorobenzene to form a 17β-alkoxycarbonyl-4-aza-5α-androst-1-en-3-one IV, (2) reacting the latter with sodium hydride under anhydrous conditions in a neutral solvent such as dimethylformamide, (3) contacting the resulting reaction mixture with an alkyl (e.g. methyl or ethyl) iodide to form the corresponding 17β-alkoxycarbonyl-4-alkyl-4-aza-5α-androst-1-en-3-one V, (4) subsequently hydrolysing the 17β-carboxy-4-alkyl-4-aza-5α-androst-1-en-3-one VI, (5) converting the steroidal acid to its corresponding 2-pyridylthio ester by refluxing with triphenyl phosphine and 2,2′-dipyridyl disulphide in an inert solvent such as toluene and isolating the resulting product, viz. 17β-(2-pyridylthiocarbonyl)-4-alkyl-4-aza-5α-androst-1-en-3-one VII by chromatography on silica gel, and (6) reacting the pyridylthio ester with an amine of formula $R^2NH$ in tetrahydrofuran to form the desired product, viz. 17β-(N-$R^2$-carbamoyl)-4-alkyl-4-aza-5α-androst-1-en-3-one (VIII), which is isolated by chromatography on silica gel.

The corresponding 17β-(N-$R^2$-carbamoyl)-4-aza-5α-androst-1-en-3-one (XIV) is readily prepared from the 17β-alkoxycarbonyl-4-aza-5α-androstone-3-one IV by repeating the above series of reaction steps but omitting Steps (2) and (3), i.e. treatment of the 4-aza-5α-androst-1-en-3-one with sodium hydride followed by methyl or ethyl iodide. This sequence of reactions goes via intermediate compounds XII and XIII.

In accordance with a further alternative process of preparing the compounds of Formula I having only hydrogen as the sole substituent on the ring A nitrogen, the double bond in the A ring is introduced as the last step of the process. Thus, a 17β-alkoxycarbonyl-4-aza-5α-androstan-3-one III is hydrolysed to the corresponding steroidal acid 17β-carboxy-4-aza-5α-androstan-3-one (IX), which in turn is converted to the corresponding pyridylthio ester, 17β-(2-pyridylthiocarbonyl)-4-aza-5α-androstan-3-one (X). This ester is treated with an amine of formula $R^2$-$NH_2$, where $R^2$ is as defined above, to form a 17β-(N-$R^2$-carbamoyl)-4-aza-5α-androstone-3-one (XI), which is dehydrogenated as previously described to product compound XIV, 17β-(N-$R^2$-carbamoyl)-4-aza-androst-1-en-3-one.

In another method, the 17β-(N-$R^2$-carbamoyl) substituent is introduced into a 17β-carboxy androstane compound of formula VI, XII or IX, by treating it in a manner similar to the procedure described in *Steroids,* Vol. 35, (3), March 1980, p. 1—7 with dicyclohexylcarbodiimide and 1-hydroxybenzotriazole to form the 17β-(1-benzotriazoloxycarbonyl)-4-aza-5α-androst-1-en-3-one or 17β-(1-benzotriazoloxycarbonyl)-4-aza-5α-androstan-3-one.

The 16-methyl derivatives, i.e. compounds in which R‴ is methyl, are prepared from known 16-methyl-17-acyl-4-methyl-4-aza-5α-androstan-3-ones, e.g. 4,16β-dimethyl-17β-acetyl-4-aza-5α-androstan-3-one, by known dehydrogenation procedures for 4-methyl-4-aza compounds to produce the corresponding 4,16β-dimethyl-17β-acetyl-4-aza-5α-androst-1-en-3-one.

The above reactions are schematically represented in the following reaction scheme, in which X is 2-pyridylthio or 1-benztriazoloxy and the other variables are as defined above.

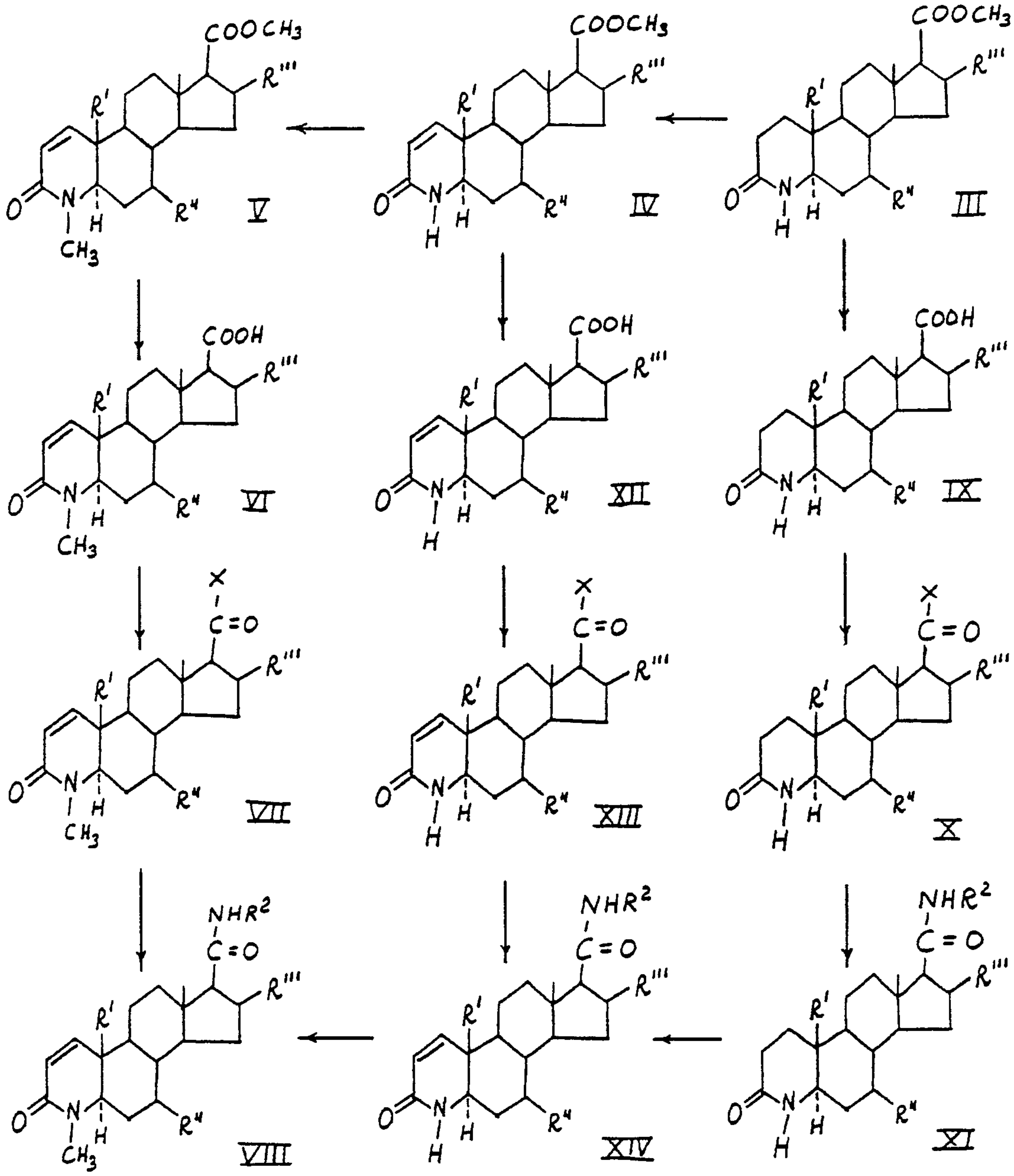

V IV III

VI XII IX

VII XIII X

VIII XIV XI

Compounds of the present invention, which may be prepared in accordance with the method described above, have been found to be potent antiandrogens by virtue of their ability to specifically inhibit testosterone-5α-reductase and are therefore suitable for treating the hyperandrogenic conditions of acne vulgaris, seborrhea and female hirsutism by topical administration, and for treating all of the above conditions, as well as benign prostatic hypertrophy, by oral parenteral administration.

The present invention thus also provides oral topical and parenteral pharmaceutical formulations for use in administering the active compounds of the present invention.

The compositions containing the compounds of the present invention as the active ingredient for use in the treatment of benign prostatic hypertrophy can be administered in a wide variety of therapeutic dosage forms in conventional vehicles for systemic administration, as, for example, by oral administration in the form of tablets, capsules, solutions or suspensions, or by intravenous injection. The daily dosage of the products may be varied over a wide range varying from 50 to 2,000 mg. The compositions are preferably provided in the form of scored tablets containing 5, 10, 25, 50, 100, 150, 250 and 500 mg. of the active ingredient for the symptomatic adjustment of the dosage to the patient to be treated. An effective amount of the drug is ordinarily supplied at a dosage level of from 1 to 50 mg/kg of body weight per day. Preferably the range is frodm 1 to 7 mg//kg. of body weight per day. These dosages are well below the toxic dose of the product. Capsules containing an active compound of this invention can be prepared by mixing it with lactose and magnesium stearate, calcium stearate, starch, talc, or other carriers, and placing the mixture in gelatin capsules. Tablets may be prepared by mixing the active ingredient with conventional

tableting ingredients such as calcium phosphate, lactose, corn starch or magnesium stearate. The liquid forms are prepared suitably flavoured suspending or dispersing agents such as the synthetic and natural gums, for example, tragacanth, acacia and methyl cellulose. Other suitable dispersing agents include glycerin. For parenteral administration, sterile suspensions and solutions are desired. Isotonic preparations, which usually contain suitable preservative, are prepared for intravenous administration.

For the treatment of acne vulgaris, seborrhoea and female hirsutism, the compounds of the present invention are administered in the form of pharmaceutical compositions comprising the active compound in combination with a pharmacologically acceptable topically administrable carrier. These topical pharmaceutical compositions may be in the form of a cream, ointment, gel or aerosol formulation suitable for application to the skin. They ordinarily include 0.1% to 15%, preferably about 5%, of the active compound, based on the total composition.

The method of preparing the novel 17β-N-(monosubstituted carbamoyl) and 17β-acyl compounds of the present invention, already described above in general terms, is further illustrated by the following Examples, some of which also describe compounds not in accordance with the invention but made by analogous methods.

Example 1

Methyl 3-oxo-4-aza-5α-androst-1-ene-17β-carboxylate

A suspension of 83.7 g of methyl 3-oxo-4-aza-5α-androstane-17-carboxylate, obtained as in U.S. Patent Specification US—A—4 377 584, and 126.5 g of benzeneseleninic anhydride in 2.09 litres of chlorbenzene was heated at reflux for 2 hours. The reflux condenser was switched to a distillation head and the mixture was distilled slowly to remove water that had formed in the reaction (2 hours). The solution was evaporated to leave 198 g of wet residue. The residue as a solution in dichloromethane was washed with saturated aqueous $NaHCO_3$ solution and saturated NaCl solution, then dried and evaporated to leave 172.4 g. This material was chromatographed on 2.56 kg of silica gel eluting first with dichloromethane (5 litres) and then with 4:1 (v/v) dichloromethane-acetone. The desired product eluted after 8 litres and amounted to 53.4 g. It was rinsed with diethyl ether and dried to leave 49.5 g., m.p. 278—280°C. This is not a compound in accordance with the invention, but, in a similar fashion, the following compounds were converted to their corresponding $\Delta^1$ derivatives:

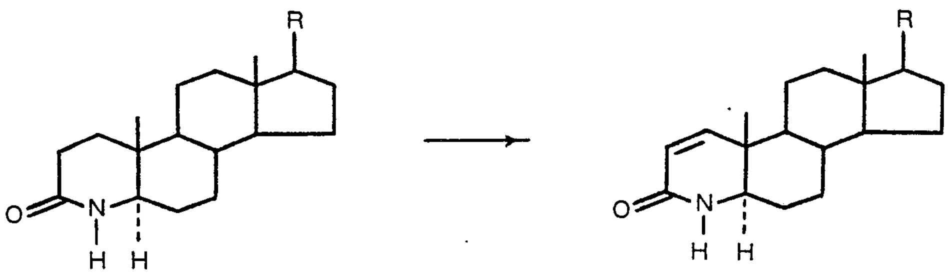

| | | m.p. |
|---|---|---|
| 1a | R = $CONHC(CH_3)_3$ | 252—254°C |
| 1b | R = $CONHC(CH_3)_2CH_2C(CH_3)_3$ | 224—226°C |

Example 2

Methyl 4-methyl-3-oxo-4-aza-5α-androst-1-ene-17β-carboxylate

A suspension of 25 g of the product of Example 1 and 2.25 g of sodium hydride in 500 ml of dry dimethylformamide was stirred under nitrogen for 15 minutes. 15 ml of methyl iodide was added dropwise and the mixture was stirred for 30 minutes at room temperature. An additional 5 ml of methyl iodide was added and the mixture was heated at 50°C for 2 hours. After cooling the mixture was diluted with water to 2 litres. The solid was separated after cooling and amounted to 25.4 g, m.p. 159—161°.

This is not a compound in accordance with the invention, but in a similar fashion, the following compounds were converted to their corresponding 4-methyl derivatives:

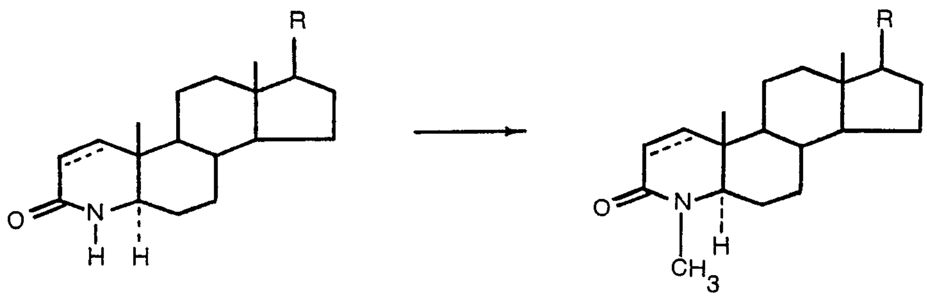

| | | m.p. |
|---|---|---|
| 2b | R = $CONHC(CH_3)_3$ | 153—155°C |
| 2c | R = $CONHC(CH_3)_2CH_2C(CH_3)_3$; | 168—170°C |

## Example 3A

S-(2-Pyridyl)4-methyl-3-oxo-4-aza-5α-androst-1-ene-17β-thiocarboxylate

A suspension of 25 g of the product of Example 2 in 125 ml of methanol was treated with a solution of KOH (12.5 g) in 12.5 ml of water. After refluxing for 4 hours, the solution was acidified with 6*N* HCl and then was diluted with water. The crude acid (23.32 g) was separated and dried. It had m.p. 300°C.

The crude dry acid (23 g), triphenylphosphine (36.45 g) and 2,2′-dipyridyldisulphide (30.4 g) was suspended in 138 ml of toluene with stirring for 3 hours at room temperature. The reaction mixture was directly chromatographed on a column of 4.5 kg of silica gel, eluting with 9:1 (v/v) ethyl acetate:acetone to give 20.4 g of the desired product, m.p. 218—220°C.

Continued elution with acetone gave 5.2 g of the methanol addition product, S-(2-pyridyl)-1α-methoxy-4-methyl-3-oxo-4-aza-5α-androstane-17β-thiocarboxylate, m.p. 221—223°C as a by-product.

3A. This is not a compound of the invention, but, in a similar fashion the product of Example 1 was converted into S-(2-pyridyl) 3-oxo-4-aza-5α-antrost-1-ene-17β-thiocarboxylate, m.. 230—232°C.

## Example 3B

N-Ethyl 4-methyl-3-oxo-4-aza-5α-androst-1-ene-17β-carboxamide

Anhydrous ethylamine was bubbled for 30 minutes into a suspension of 2.5 g of the pyridylthioester of Part A in 70 ml of tetrahydrofuran. After 60 minutes exposure, the resulting solution was evaporated and the residue was chromatographed on 125 g of silica gel. Elution with 20:1 ethyl acetate/dichloromethane afforded 1.5 g of the product, m.p. 240—242°C.

This is not a compound in accordance with the invention, but, when the example is repeated using an appropriate amine and an appropriate pyridylthioester, the following products were obtained:

3a: N-*t*-butyl-4-methyl-3-oxo-4-aza-5α-androst-1-ene-17β-carboxamide, m.p. 152—154°C.

3b: N-(2,4,4-trimethyl-2-pentyl)-4-methyl-3-oxo-4-aza-5α-androst-1-ene-17β-carboxamide, m.p. 168—170°C.

## Example 4A

3-Oxo-3,5-secoetiocholane-3,20-dioic acid

To a solution of 200 g of 3-oxo-4-etiocholen-20-oic acid in 3.5 l of *t*-butanol at 80° was added a solution of 198.4 g of sodium carbonate in 474 ml of water. A warm (65°C) solution of 948.5 g of sodium metaperiodate and 6.95 g of potassium permanganate in 3.5 l of water was added at such a rate that the reaction mixture was maintained at 80°C. After addition the mixture was heated at reflux for one hour. The mixture stood at room temperature overnight. The inorganic salts were removed by filtration and the cake was washed with 225 ml of water. A solution of 5% aqueous sodium bisulphite was added to reduce the iodine that was present. The *t*-butanol was removed under reduced pressure and the aqueous residue was acidified with concentrated hydrochloric acid. The separated gum was extracted into dichloromethane and was washed with 5% aqueous sodium bisulphite and saturated sodium chloride solution, then dried and concentrated to an off-white residue (214 g). Crystalline material was obtained by suspending the residue in ether and diluting with hexane to give 152 m.p. 189—192°C.

## Example 4B

3-Oxo-4-aza-5-etiocholen-20-oic acid

A suspension of 64.7 g of the dioic acid or Step A in 350 ml of ethylene glycol was treated with 80 ml of liquid ammonia. The resulting solution was heated at a rate of 3°/min. up to 180°C and was held at that temperature for 15 minutes. After cooling, 1 litre of water was added and the mixture was acidified with 10% hydrochloric acid to a pH of 1.5. The product was removed and washed with water, then air dried to leave 57.5 g of the product, m.p. 310°C.

Example 4C

3-Oxo-4-aza-5α-etiocholan-20-oic acid

A solution of 136 g of the $\Delta^5$-acid of Example 4B in 16.32 ml of acetic acid was hydrogenated at 60°C in the presence of platinum catalyst (from 16.32 g of $PtO_2$) at 40 psig (276 kPa over the atmospheric pressure) for 3 hours. The catalyst was removed and the solution concentrated to give 128.2 g of crude product. The material was washed well with 3 l of water, then filtered and air dried to leave 125 g of the white solid, m.p. 310°.

This material was also obtained by saponification of methyl 3-oxo-4-aza-5α-androstane-17β-carboxylate (methyl 3-oxo-4-aza-5α-etiocholan-20-oate) in 7% methanolic potassium hydroxide followed by an acidic work-up.

Example 4D

N-(2,4,4-trimethyl-2-pentyl) 3-oxo-4-aza-5α androstane-17β-carboxamide

A solution of 5.0 g of the product 4C, 3.35 g of dicyclohexylcarbodiimide and 3.18 g of 1-hydroxybenztriazole in 500 ml of dichloromethane was stirred at room temperature overnight. The solid was separated by filtration and the filtrate was treated with 2,4,4-trimethyl-2-pentylamine (*t*-octylamine). This solution stood at room temperature for 64 hours. A small amount of solid was removed and the solution was washed successively with 10% (w/v) aqueous sodium hydroxide, water, 10% (w/v) hydrochloric acid and saturated aqueous sodium chloride. After drying and concentration the crude product was eluted through 240 g of silica gel with 3:7 (v/v) acetone-dichloromethane to give 5.5 g of the product, m.p. 250—251°C.

Example 4E

Example 4D is repeated using *t*-butylamine in place of 2,2,4-trimethyl-2-pentylamine to obtain N-*t*-butyl-3-oxo-4-aza-5α-androstane-17β-carboxamide, m.p. 274—276°C.

To a solution of 7.2 g of S-(2-pyridyl)-3-oxo-4-aza-5α-androst-1-ene-17β-thiocarboxylate in 288 ml of tetrahydrofuran was added at −78°C 33.6 ml of 1.3M S-butylmagnesium chloride. After 30 minutes at −78°C the solution was allowed to reach room temperature and was then treated with saturated aqueous NaCl solution. The product was extracted into dichloromethane and was washed with saturated aqueous NaCl solution and 10% aqueous NaOH solution, then dried and concentrated. The residue was eluted through a 430 g of silica gel with 9:1 (v/v) dichloromethane-acetone to give 4.5 g of the product, m.p. 246—249°C.

When the procedure is repeated using the following reagents, the indicated product is obtained.

| Starting material | Reagent | product |
|---|---|---|
| S-(2-pyridyl)3-oxo-4-methyl-5α-androst-1-ene-17β-thiocarboxylate | sec-butyl magnesium chloride | 4,22-dimethyl-4-aza-21-nor-5α-chol-1-ene-3-one m.p. 134-136°C |
| S-(2-pyridyl)3-oxo-4-aza-5α-androstene-17β-thiocarboxylate | isobutyl magnesium chloride | 23-methyl-4-aza-21-nor-5α-cholane-3,20-dione m.p. 220—222°C. |

Example 7

22-Methyl-4-aza-21-nor-5α-chol-1-ene-3,20-dione (second method)

A solution of 21 g of 22-methyl-4-aza-21-nor-5α-cholane-3,20-dione (Step 1) and 29.49 g of benzeneseleninic anhydride in 552 ml of chlorobenzene was refluxed with water separation for 4 hours. The mixture was concentrated and the residue was redissolved in dichloromethane. After washing with 10% (w/v) aqueous sodium hydroxide, 10% (w/v) hydrochloric acid and saturated aqueous sodium hydroxide, the solution was dried and concentrated to 45 g of yellow residue. This was chromatographed on 1.5 kg of silica gel packed in dichloromethane and eluted with ethyl acetate to give 10.6 g of the product, m.p. 248—251°C.

When the procedure is repeated using 23-methyl-4-aza-21-nor-5α-cholane-3,20-dione as starting material, the product obtained is 23-methyl-4-aza-21-nor-5α-chol-1-ene-3,20-dione, m.p. 283—296°C.

**Claims for the Contracting States: BE CH/LI DE FR GB IT LI LU NL SE**

1. A compound of the formula:

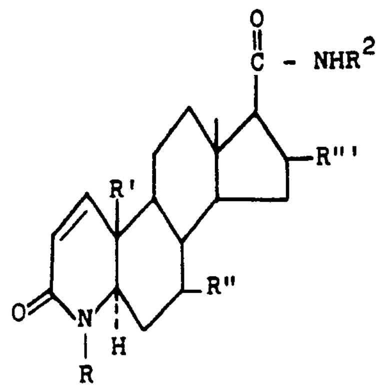

I

in which R is hydrogen, methyl or ethyl;

$R^2$ is a tertiary alkyl radical having up to 12 carbon atoms and having a tertiary carbon atom directly bonded to the carbamoyl nitrogen atom;

R′ is hydrogen or methyl;

R″ is hydrogen or β-methyl, and

R‴ is hydrogen, α-methyl or β-methyl.

2. A compound of Claim 1 having the formula:

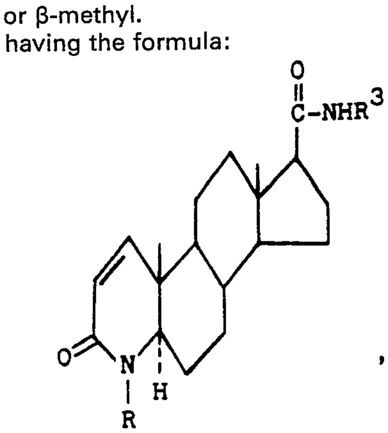

,

II

in which R is hydrogen, methyl or ethyl and $R^3$ is tertiary alkyl of from 4-8 carbon atoms and having a tertiary carbon atom directly bonded to the carbamoyl nitrogen atom.

3. 17β-(N-*t*-butylcarbamoyl)-4-methyl-4-aza-androst-1-en-3-one.

4. 17β(N-*t*-butylcarbamoyl)-4-aza-androst-1-en-3-one.

5. A compound as claimed in any one of Claims 1 to 7 for use in treating one or more of the hyperandrogenic conditions of acne vulgaris, seborrhea, female hirsutism, and benign prostatic hypertrophy by oral, parenteral or topical administration.

6. A pharmaceutical composition comprising a pharmaceutical acceptable carrier and a compound as claimed in any one of Claims 1 to 4.

**Claims for the Contracting State: AT**

1. A method of preparing a compound of the formula:

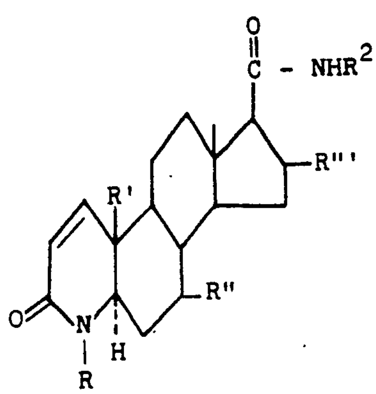

I

in which R is hydrogen, methyl or ethyl;

$R^2$ is a tertiary radical having up to 12 carbon atoms and having a tertiary carbon atom directly bonded to the carbamoyl nitrogen atom;

R′ is hydrogen or methyl;

R″ is hydrogen or β-methyl, and

R‴ is hydrogen, α-methyl or β-methyl,

comprising reacting a compound of formula:

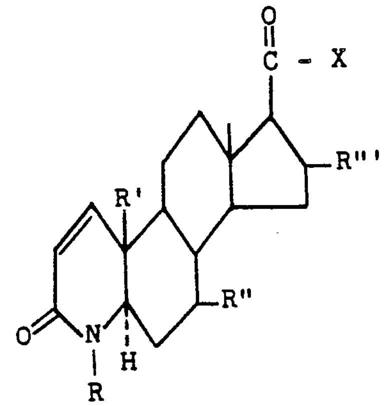

,

in which X is 2-pyridylthio, with an amine $NHR^2$ in tetrahydrofuran.

2. A method as claimed in Claim 1 in which the starting compound has been prepared by refluxing a compound of formula:

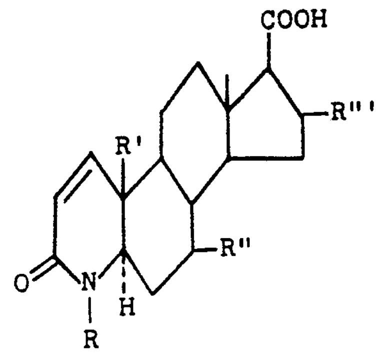

,

where the symbols are as defined in Claim 1 with triphenylphosphine and 2,2′-dipridyl disulphide in an inert solvent.

3. A method of preparing a compound of the formula:

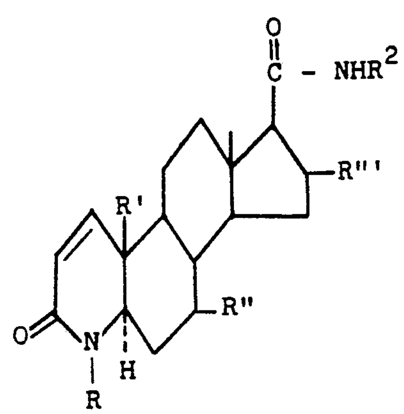

I ,

in which R is hydrogen, methyl or ethyl;

$R^2$ is tertiary alkyl radical having up to 12 carbon atoms and having a tertiary carbon atom directly bonded to the carbamoyl nitrogen atom;

R′ is hydrogen or methyl;

R″ is hydrogen or β-methyl, and

R‴ is hydrogen, α-methyl or β-methyl,

comprising reacting a compound of formula:

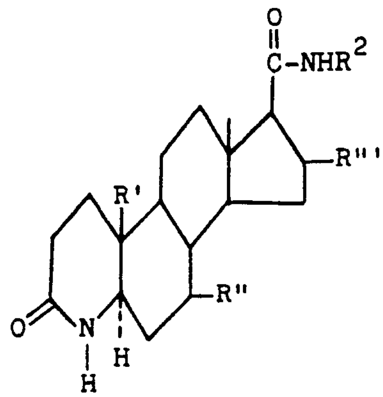

,

in which the symbols are as defined in Claim 1 with a dehydrogenating agent.

4. A method as claimed in Claim 3 in which the starting compound has been prepared by reacting a compound of formula:

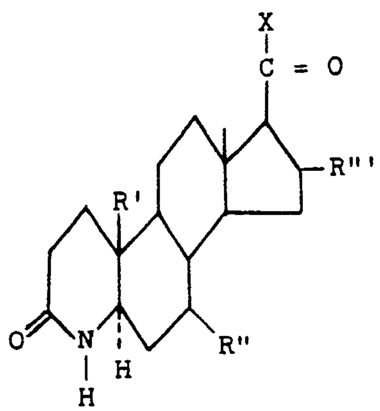

X

with an amine $NHR^2$ in tetrahydrofuran.

5. A method as claimed in any one of Claims 1 to 4 as applied to the preparation of a compound having the formula:

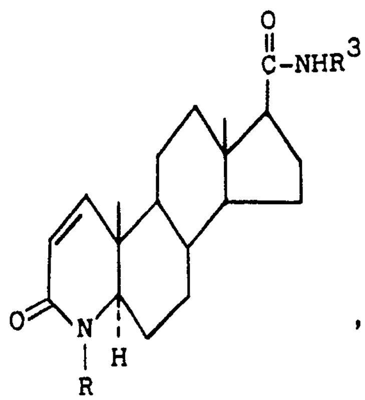

II

,

in which R is hydrogen, methyl or ethyl and $R^3$ is tertiary alkyl of from 4-8 carbon atoms and having a tertiary carbon atom directly bonded to the carbamoyl nitrogen atom.

6. A method as claimed in any one of Claims 1 to 4 as applied to the preparation of 17β-(N-*t*-butylcarbamoyl)-4-methyl-4-aza-androst-1-en-3-one.

7. A method as claimed in any one of Claims 1 to 4 as applied to the preparation of 17β-(N-*t*-butylcarbamoyl)-4-aza-androst-1-en-3-one.

**Patentansprüche für die Vertragsstaaten: BE CH/LI DE FR GB IT LI LU NL SE**

1. Verbindung der Formel

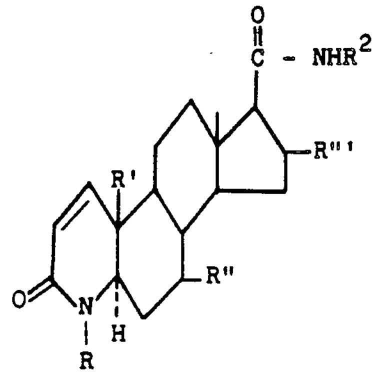

I

worin
R Wasserstoff, Methyl oder Ethyl ist;
$R^2$ ein tertiärer Alkylrest mit bis zu 12 Kohlenstoffatomen und einem direkt an das Carbamoyl-Stickstoffatom gebundenen tertiären Kohlenstoffatom ist;
R′ Wasserstoff oder Methyl ist;
R″ Wasserstoff oder β-Methyl ist; und
R‴ Wasserstoff, α-Methyl oder β-Methyl ist.

2. Verbindung nach Anspruch 1 mit der Formel

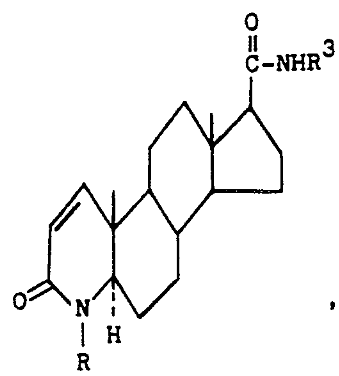

II

,

worin R Wasserstoff, Methyl oder Ethyl ist und $R^3$ ein tertiäres Alkyl mit 4 bis 8 Kohlenstoffatomen und einem direkt an das Carbamoyl-Stickstoffatom gebundenen tertiären Kohlenstoffatom ist.

3. 17-β-(N-*t*-Butylcarbamoyl)-4-methyl-4-aza-adrost-1-en-3-on.

4. 17-β-(N-*t*-Butylcarbamoyl)-4-aza-androst-1-en-3-on.

5. Verbindung, wie in einem der Ansprüche 1 bis 4 beansprucht, zur Verwendung bei der Behandlung eines oder mehrerer der hyperandrogenen Zustände von Akne vulgaris, Seborrhoea, Hirsutismus der Frau und gutartige Hypertrophie der Prostata durch orale, parenterale oder topische Verabreichung.

6. Pharmazeutische Zusammensetzung, welche einen pharmazeutisch annehmbaren Träger und eine Verbindung, wie in einem der Ansprüche 1 bis 4 beansprucht, umfasst.

**Patentansprüche für den Vertragsstaat: AT**

1. Ein Verfahren zur Herstellung einer Verbindung der Formel:

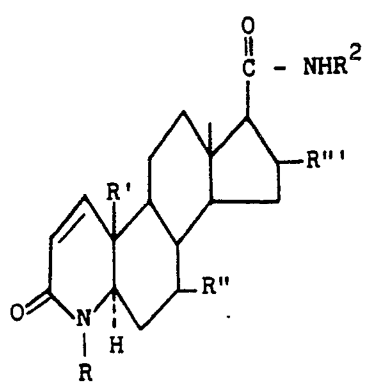

I ,

worin

R Wasserstoff, Methyl oder Ethyl ist;

$R^2$ ein tertiärer Alkylrest mit bis zu 12 Kohlenstoffatomen ist, der ein tertiäres Kohlenstoffatom direkt an das Carbamoyl-Stickstoffatom gebunden aufeweist;

R' Wasserstoff oder Methyl ist;

R'' Wasserstoff oder β-Methyl ist; und

R''' Wasserstoff, α-Methyl oder β-Methyl ist,

umfassend das Umsetzen einer Verbindung der Formel:

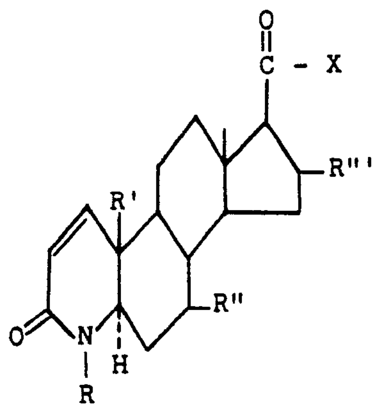

,

in welcher X 2-Pyridylthio ist, mit einem Amin $NHR^2$ in Tetrahydrofuran.

2. Ein Verfahren wie in Anspruch 1 beansprucht, bei welchem die Ausgangsverbindung durch Erhitzen einer Verbindung der Formel:

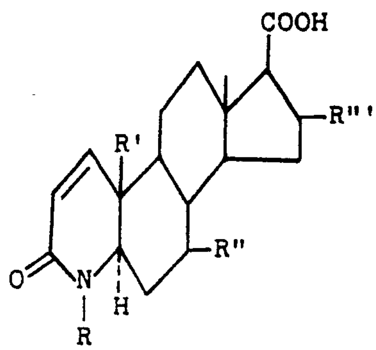

,

worin die Symbole wie in Anspruch 1 definiert sind, mit Triphenylphosphin und 2,2'-Dipyridyldisulfid unter Rückfluß in einem inerten Lösungsmittel hergestellt worden ist.

3 Ein Verfahren zur Herstellung einer Verbindung der Formel:

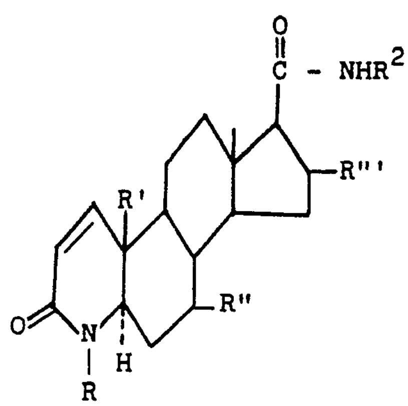

I

worin

R Wasserstoff, Methyl oder Ethyl ist;

$R^2$ ein tertiärer Alkylrest mit bis zu 12 Kohlenstoffatomen ist, der ein tertiäres Kohlenstoffatom direkt an das Carbamoyl-Stickstoffatom gebunden aufweist;

R' Wasserstoff oder Methyl ist;

R'' Wasserstoff oder β-Methyl ist; und

R''' Wasserstoff, α-Methyl oder β-Methyl ist,

umfassend das Umsetzen einer Verbindung der Formel:

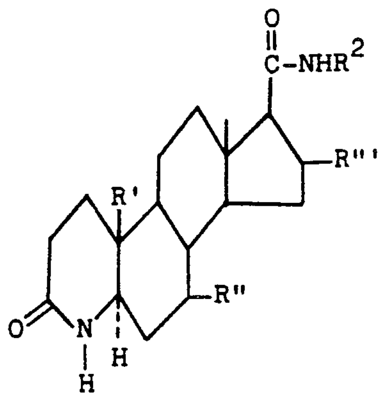

,

in welcher die Symbole wie in Anspruch 1 definiert sind, mit einem Dehydrogenierungsmittel.

4. Ein Verfahren wie in Anspruch 3 beansprucht, wobei die Ausgangsverbindung durch Umsetzen einer Verbindung der Formel:

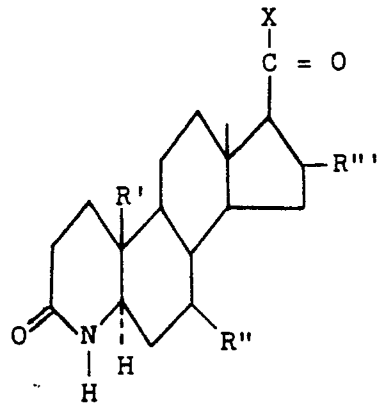

X

mit einem Amin $NHR^2$ in Tetrahydrofuran hergestell worden ist.

5. Ein Verfahren wie in einem der Ansprüche 1 bis 4 beansprucht, angewendet auf die Herstellung einer Verbindung der Formel:

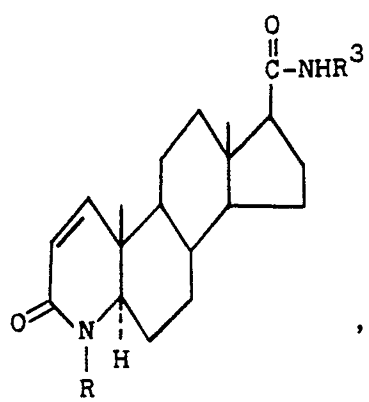

II

,

in welcher R Wasserstoff, Methyl oder Ethyl ist, und $R^3$ Tertiäralkyl mit 4—8 Kohlenstoffatomen ist, das ein tertiäres Kohlenstoffatom direkt an das Carbamoylstickstoffatom gebunden aufweist.

6. Ein Verfahren wie in einem der Ansprüche 1 bis 4 beansprucht, angewendet bei der Herstellung von 17β-(N-tert.Butylcarbamoyl)-4-methyl-4-aza-androst-1-en-3-on.

7. Ein Verfahren wie in einem der Ansprüche 1 bis 4 beansprucht, angewendet bei der Herstellung von 17β-(N-tert.-Butylcarbamoyl;-4-aza-androst-1-en-3-on.

**Revendications pour les Etats contractants: BE CH/LI DE FR GB IT LI LU NL SE**

1. Composé de formule

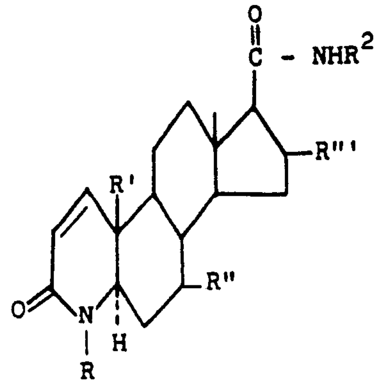

I

dans laquelle R est l'hydrogène, le méthyle ou l'éthyle;

$R^2$ est un radical alkyle tertiaire contenant jusqu'à 12 atomes de carbone et ayant un atome de carbone tertiaire lié directement à l'atome d'azote carbamoyle;

R' est l'hydrogène ou le méthyle;

R'' est l'hydrogène ou le β-méthyle; et

R''' est l'hydrogène, l'α-méthyle ou la β-méthyle.

2. Composé selon la revendication 1 ayant la formule

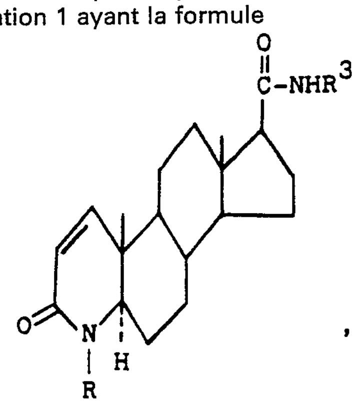

II

,

dans laquelle R est l'hydrogène, le méthyle ou l'éthyle et $R^3$ est un alkyle tertiaire de 4—8 atomes de carbone et ayant un atome de carbone tertiaire directement lié à l'atome d'azote carbamoyle.

3. 17β-(N-*t*-butylcarbamoyl)-4-méthyl-4-aza-androstène-1-one-3.

4. 17β-(N-*t*-butylcarbamoyl)-4-aza-androstène-1-one-3.

5. Composé selon l'une quelconque des revendications 1 à 7 pour l'utilisation dans le traitement de un ou plusieurs états hyper-androgèniques tels que l'acné vulgaris, la séborrhée, l'hursutisme féminin, l'hypertrophie bénigne de la prostate par administration orale, parentèrale ou topique.

6. Composition pharmaceutique comprenant un véhicule pharmaceutiquement acceptable et composé selon l'une quelconque des revendications 1 à 4.

**Revendications pour l'Etat Contractant: AT**

1. Procédé de préparation d'un composé de formule:

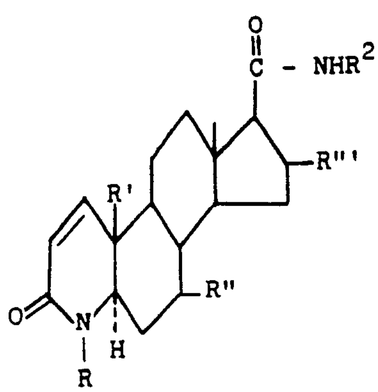

I

dans laquelle R est l'hydrogène, le méthyle ou l'éthyle;

$R^2$ est un radical alkyle tertiaire contenant jusqu'à 12 atomes de carbone et ayant un atome de carbone tertiaire lié directement à l'atome d'azote carbamoyle;

R' est l'hydrogène ou le méthyle;

R'' est l'hydrogène ou le β-méthyle; et

R''' est l'hydrogène, l'α-méthyle ou la β-méthyle,

qui comprend de faire réagir un compose de formule:

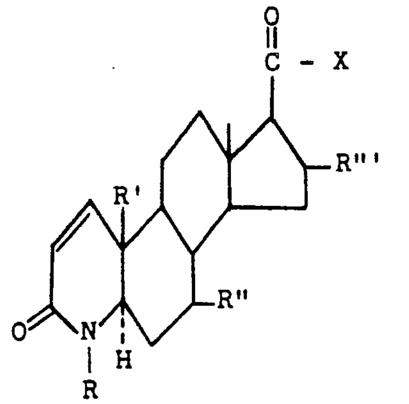

,

dans laquelle X est le 2-pyridylthio, avec une amine $NHR^2$ dans le tétrahydrofuranne.

2. Procédé selon la revendication 1 dans lequel le composé de départ a été préparé en chauffant à reflux un composé de formule:

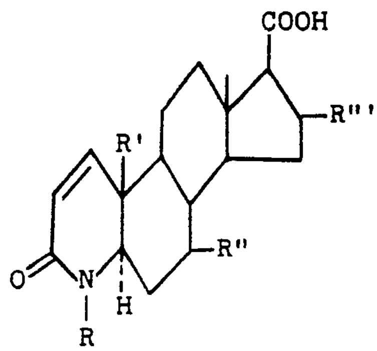

,

dans laquelle les symboles sont comme définis dans la revendication 1 avec de la triphénylphosphine et du disulfure de 2,2'-dipyridyle dans un solvant inerte.

3. Procédé de préparation d'un composé de formule:

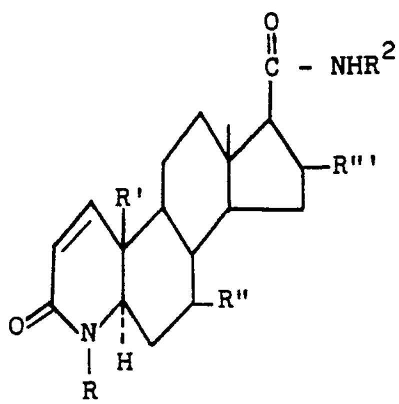

I

dans laquelle R est l'hydrogène, le méthyle ou l'éthyle;

$R^2$ est un radical alkyle tertiaire contenant jusqu'à 12 atomes de carbone et ayant un atome de carbone tertiaire lié directement à l'atome d'azote carbamoyle;

R' est l'hydrogène ou le méthyle;

R'' est l'hydrogène ou le β-méthyle; et

R''' est l'hydrogène, l'α-méthyle ou le β-méthyle,

qui comprend de faire réagir un composé de formule

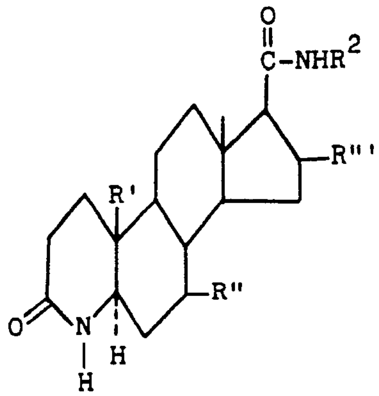

,

dans laquelle les symboles sont comme définis dans la revendication 1 avec un agent deshydrogènant.

4. Procédé selon la revendication 3 dans lequel le composé de départ a été préparé en faisant réagir un composé de formule

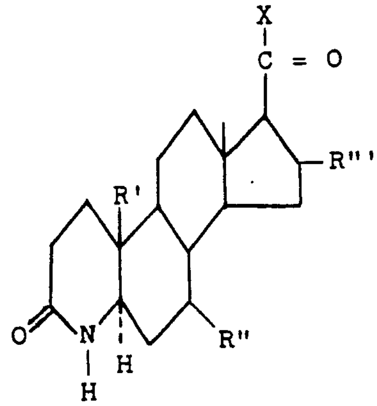

X

avec une amine $NHR^2$ dans le tétrahydrofuranne.

5. Procédé selon l'une quelconque des revendication 1 à 4 pour préparer un composé ayant la formule:

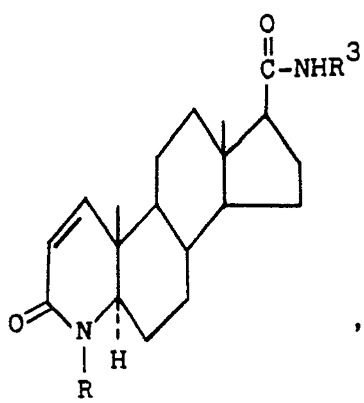

II

,

dans laquelle R est l'hydrogène, le méthyle ou l'éthyle et $R^3$ est un alkyle tertiaire de 4—8 atomes de carbone et ayant un atome de carbone tertiaire directement lié à l'atome d'azote carbamoyle.

6. Procédé selon l'une quelconque des revendication 1 à 4 appliqué à la préparation de la 17β-(N-*t*-butylcarbamoyl)-4-méthyl-4-aza-androstène-1-one-3.

7. Procédé selon l'une quelconque des revendication 1 à 4 appliqué à la préparation de la 17β-(N-*t*-butylcarbamoyl)-4-aza-androstène-1-one-3.